# EUROPEAN PATENT APPLICATION

(11) **EP 0 614 967 A2**
(43) Date of publication of application: **14.09.1994**
(21) Application number: 94301247.6
(22) Date of filing: 22.02.1994
(51) Int. Cl.: C12M 3/04

(54) **Three piece roller bottle assembly for culturing cells having multiple longitudinal elements on the interior surface**

(30) Priority: 09.03.1993 US 28448
(71) Applicant: Becton Dickinson and Company, Franklin Lakes, New Jersey 07417-1880 (US)
(72) Inventor: Mussi, Edward F., Hewitt, New Jersey 07421 (US); Gray, Harry Edward, Bloomingdale, New Jersey 07403 (US); Fedun, Oresta Natalia, Wanaque, New Jersey 07465 (US)
(74) Representative: Ruffles, Graham Keith

(57) **Abstract**

A roller bottle assembly for cell growth culturing of the present invention includes a generally cylindrical bottle with a longitudinal axis. The bottle includes a bottom portion, a sidewall portion and a top portion which define a hollow chamber. The bottom portion is fluidly tightly attached to the sidewall portion at a first end perpendicular to the longitudinal axis. The top portion is fluidly tightly attached to the sidewall portion at a second end perpendicular to the longitudinal axis. The top portion includes a provision for introducing liquid into the chamber. The sidewall portion has an inner and outer surface, with the inner surface having a multiplicity of longitudinal elements integrally formed thereon having portions with elevations above and below an average elevation of the surface which serve as attachment surfaces for growing cells.

## Description

### Background of the Invention

### 1. Field of the Invention

The present invention relates to a roller bottle assembly for cell growth production, and more particularly relates to a roller bottle with an interior surface which enhances cell attachment and growth.

### 2. Description of Related Information

Containers used in laboratory settings for cell culturing are commonly known as "roller bottles." These roller bottles are generally cylindrical and are adapted to rotate about their longitudinal axis when placed on a laboratory roller apparatus. The laboratory roller apparatus is often adapted to fit within chambers for controlling the temperature and atmosphere. The bottles have evolved into a standard size of about 0.85, 1.75 and 3.5 liters capacity and about 11-12 cm. in diameter because of the constraints of the roller apparatus.

The most common application for these bottles is to be charged with a few hundred ml of growth medium containing a suspension of the cells to be cultured, placed on their sides on a roller apparatus so that a longitudinal portion of the inside surface of the bottle is always wet by the growth medium, and rotated slowly (about 1-2 rpm) for a number of days. During this time, the cells grow and utilize the nutrients present in the growth medium, generally by attaching themselves to the surface of the bottle. In many applications, it is necessary to drain and recharge the growth medium during the course of the growth cycle, either to harvest by-products, remove waste or replenish nutrients.

The roller bottles are used in research and in commercial and industrial labs. In most of these applications, users are interested in growing large numbers of cells, generally to collect cell by-products. Thus anything which will increase the numbers of cells, and enhance the yield of the desired product, is a benefit.

One solution to the problem would be to simply increase the size of the bottle. However, given the general standardization of roller apparatus in the field, this does not answer many requirements. Some manufacturers do offer bottles having the same diameter in several length versions which reduce the amount of handling when running larger scale programs, but these bottles may not fit some apparatus and may present handling problems.

Since in many of the cell growth applications, bottles need to be handled several times during the growth cycle, it has been recognized that increasing the inside surface area of the bottle, with a concomitant increase in the number of cells, will increase the amount of the desired product per amount of space taken up and time spent handling bottles.

United States Patent 4,317,886 teaches placing one or more hollow concentric tubes within the bottle providing a substantial increase in surface area within the same exterior dimension. The '886 design, while workable, could be somewhat complex to manufacture and currently is not commercially available. Others have provided teachings to increase available surface area. United States Patent No. 5,010,013 teaches a roller bottle having increased surface area.

The '013 bottle is manufactured in a single step as a unitary piece and increases the interior surface area with a series of baffles or accordion-like pleats which form the sidewall of the bottle. These pleats are perpendicular to the bottle axis. United States Patents, 4,962,033 and Des. 318,800 relate to bottles having the pleated accordion-like design. They also include longitudinal drainage channels which help to strengthen the bottles and facilitate drainage during media changes. Problems which may exist with these type bottles may include breakage at the pleat points, and longitudinal flexibility during handling. Further, the pleats are large with respect to the bottle wall thickness and bottles with this design must be charged with about 50 to 100 percent more growth medium than equivalent sized smooth wall bottles to achieve coverage of the pleat surface.

United States Patent 5,084,393 teaches increasing the interior surface area of a roller bottle by lining the interior walls with a plurality of bristle shaped projections. While bristle shaped projections as taught in '393 greatly increase the surface area, actual manufacture, which may include a central holder section for the bristles, is complex.

If an easily manufactured roller bottle having an increased internal surface area which could be produced with efficiencies similar to smooth wall bottles were available, an advance to the cell culturing arts would be made. Such a roller bottle is achieved by the present invention.

### Summary of the Invention

A roller bottle assembly for cell growth culturing of the present invention includes a generally cylindrical bottle with a longitudinal axis having a bottom portion, a sidewall portion having a first end and a second end, and a top portion. The portions define a hollow chamber with the bottom portion being fluidly tightly attached to the sidewall portion at the first end and the top portion being fluidly tightly attached to the second end of the sidewall. Both the top portion and the bottom portion are generally perpendicular to the axis of the bottle. The top portion has a provision for introducing fluid into the bottle. The sidewall portion of the bottle has an inner and an outer surface. The inner surface has a multiplicity of longitudinal elements preferably integrally formed thereon which serve as attachment surfaces for growing cells. The longitudinal elements have a first portion with an elevation above an average elevation of the inside surface and a second portion with an elevation below an average elevation of the inside surface. The longitudinal elements also include at least one substantially optically transparent section to allow inspection of cells on the inner surface.

The present invention increases the inside surface area of a roller bottle with no increase in exterior size. Bottles of the instant invention in side-by-side comparisons with commercially available bottles of similar size having smooth inner surfaces provide a statistically significant increase in numbers of cells growing under equivalent conditions with the same amount of growth medium. The increased surface area of the present invention allows a practitioner to grow significantly more cells using the same amount of reagents, laboratory space and time, thus providing an advance to the art of cell growth culturing.

### Brief Description of the Drawings

Fig. 1 is a cutaway perspective view of a roller bottle assembly of the present invention;
Fig. 2 is a cross-section of the sidewall portion of the roller bottle of the present invention taken along the line 2,2;
Fig. 3 is an enlarged cross-sectional detail of a portion of the sidewall portion of the roller bottle of Fig. 2;
Fig. 4 is an enlarged schematic of a cross-section of the elongate members from the sidewall portion of the present invention;
Fig. 5 is a perspective view of the roller bottle assembly of the present invention in use on a laboratory roller apparatus; and
Fig. 6a-6i are schematics from photo-micrographs of cross-sections of the sidewall portion of the present invention.

### Detailed Description

While this invention is satisfied by embodiments in many different forms, there is shown in the drawings and will herein be described in detail a preferred embodiment of the invention, with the understanding that the present disclosure is to be considered exemplary of the principles of the invention and is not intended to limit the invention to the embodiment illustrated. The scope of the invention will be measured by the appended claims and their equivalents.

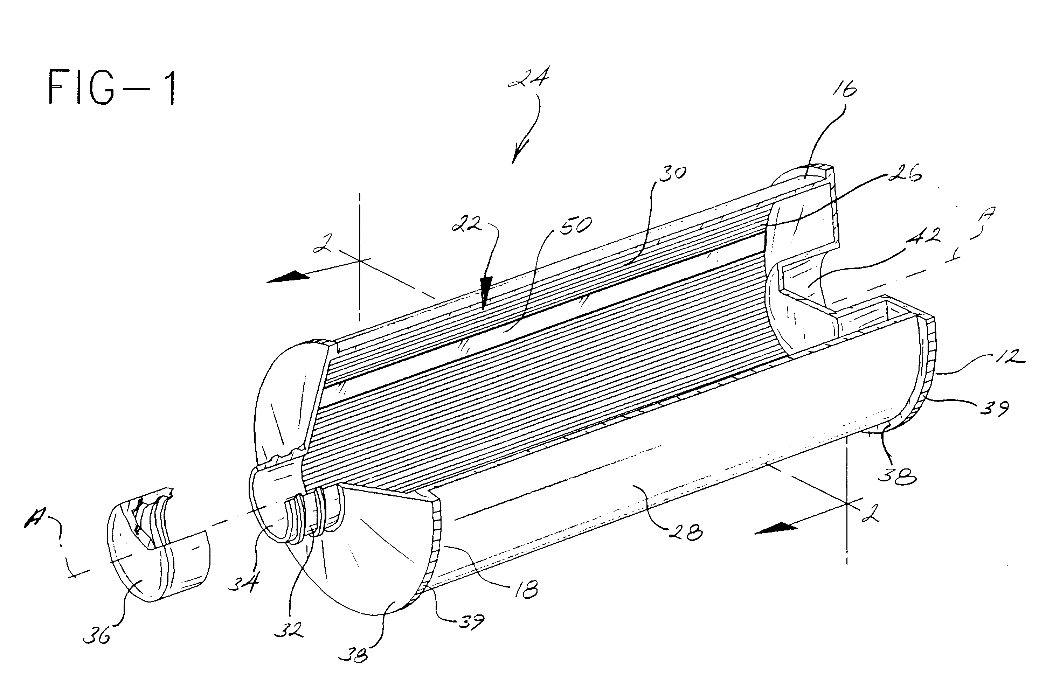

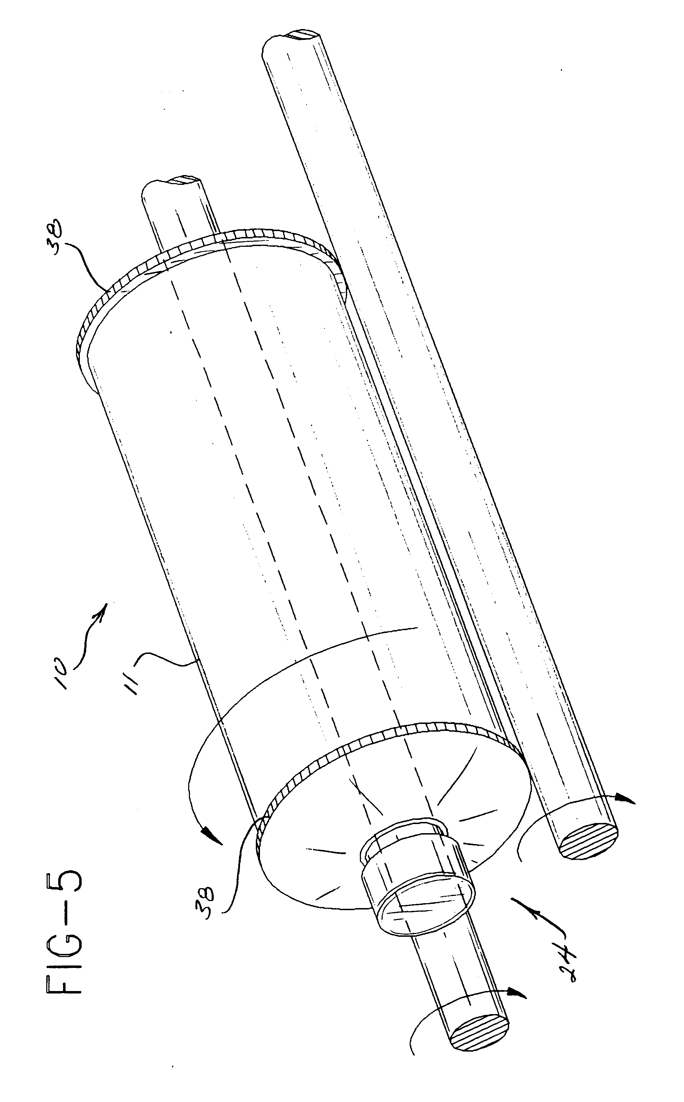

Referring to the drawings, particularly Figs. 1-4, a roller bottle assembly for cell growth culturing 10 includes a roller bottle 11 having a longitudinal axis A, a bottom portion 12, a sidewall portion 14 having a first end 16, a second end 18 and a top portion 20. The portions define a hollow chamber 22. Bottom portion 12 is fluidly tightly attached to sidewall portion 14 at first end 16 generally perpendicular to axis A. Top portion 20 has a covered opening 24 for introducing liquid into chamber 22.

Sidewall portion 14 has an inner surface 26 and an outer surface 28. The inner surface 26 has a multiplicity of longitudinal elements 30 preferably integrally formed thereon which serve as attachment surfaces for growing cells.

The top portion covered opening 24 for introducing liquid preferably includes an axially positioned neck 32 with an opening 34 to chamber 22. Neck 32 preferably has a removable conjugate cap 36 for sealing opening 34. While neck 32 and conjugate cap 36 may be threaded or fitted with other suitable provisions for sealing and opening such as a snap-cap or the like, preferably cap 36 and neck 32 are threaded, so that the cap 36 may be left partially opened so as to allow interchange of gases between chamber 22 and an outside environment.

Adverting to Fig. 5, roller bottle assembly 10 preferably includes annular rings 38 with serrations 39 on top portion 20 and bottom portion 12 for positioning roller bottle 11 on a laboratory roller system 40. One skilled in the art of laboratory rollers and roller bottles will appreciate that rings 38 also would allow an additional roller bottle of like construction to be placed on top of bottles of the present invention placed on adjacent racks (not shown).

Bottom portion 12 of the roller bottle may include a central concave portion 42 sized to partially accommodate the cap and neck portion of an adjacent bottle assembly 10 similarly longitudinally oriented with neck 32 and cap 38, thereby allowing closer placement of bottles on laboratory rack system 40.

Inner surface 26 may have a surface treatment to enhance the facility with which certain cells will attach to surface 26, thereby enhancing the yield. Such treatments may include plasma treatment, corona discharge treatment, surface oxidation and the like.

Bottom portion 12 and top portion 20 preferably are attached to first end 16 and second end 18 of sidewall portion 14 respectively, by methods including adhesive bonding, solvent bonding, spin welding, ultrasonic welding, mechanical interference fitting or any other method which results in a fluid tight seal of sufficient mechanical strength to provide functionality.

Roller bottle 11 preferably is formed from a plastic resin which may be a thermoplastic including polypropylene, polycarbonate, polyurethane, polyvinyl chloride, polymethylmethacrylate, polystyrene, polyethyleneterephthalate, polyester and most preferably has sidewall portion 14 extruded from crystalline polystyrene.

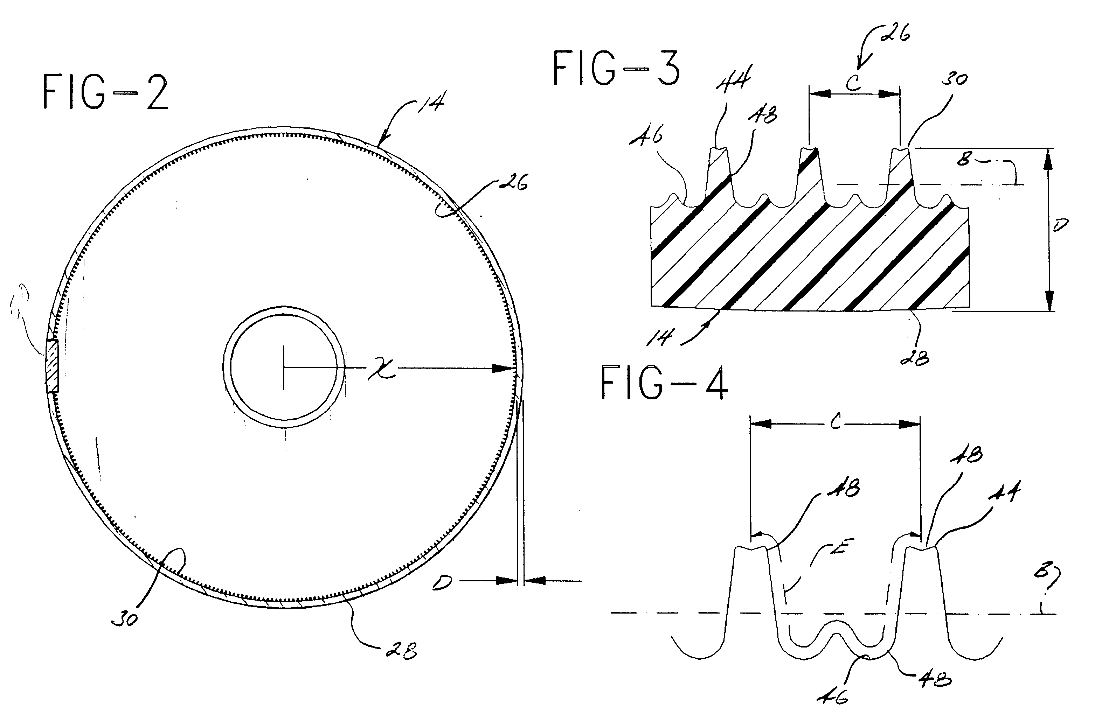

Adverting to Figs. 2, 3 and 4, longitudinal elements 30 include a longitudinal area first portion 44 having elevations above an average surface elevation B of inside surface 26 and second portion 46 having elevations below the average surface elevation B of inside surface 26. Longitudinal elements 30 further include substantially smooth transitions 48 between first and second portions 44 and 46. Longitudinal elements 30 also include at least one longitudinal element 50 which allows microscopic inspection of cells on the inside surface of the bottle from outside the bottle. In the preferred embodiment, sidewall portion 14 is preferably extruded as tubing from crystalline polystyrene, then cut to length, thus element 50 can be formed substantially optically transparent. This is contrasted to other bottles in the art which are blow molded, often from materials which are not substantially transparent.

Longitudinal elements 30, with portions 44 and 46 having elevations above and below the average surface elevation B and a pitch C between like elements, are small compared to a radius R of sidewall portion 14 and additionally represent only a fraction of an overall thickness D of sidewall 14. Preferably elevations of first portion 44 above average elevation B and second portion 46 below average elevation B of inner surface 26 of sidewall portion 14, are within the range of about 1.3mm to about 0.1mm and most preferably first portion 44 is about 0.6mm above and second portion 46 is about 0.2mm below average elevation B. Preferably, pitch C, the distance between similar elements of similar elevations, is about 0.5mm to about 1.6mm, and most preferably about 1.1mm. Radius X of sidewall portion 14 is preferably about 5 to 6cm, most preferably about 5.67 to about 5.80cm. Sidewall portion 14 has thickness D which preferably is about 1.6mm to about 2.5mm and most preferably about 1.8mm to about 2.2mm.

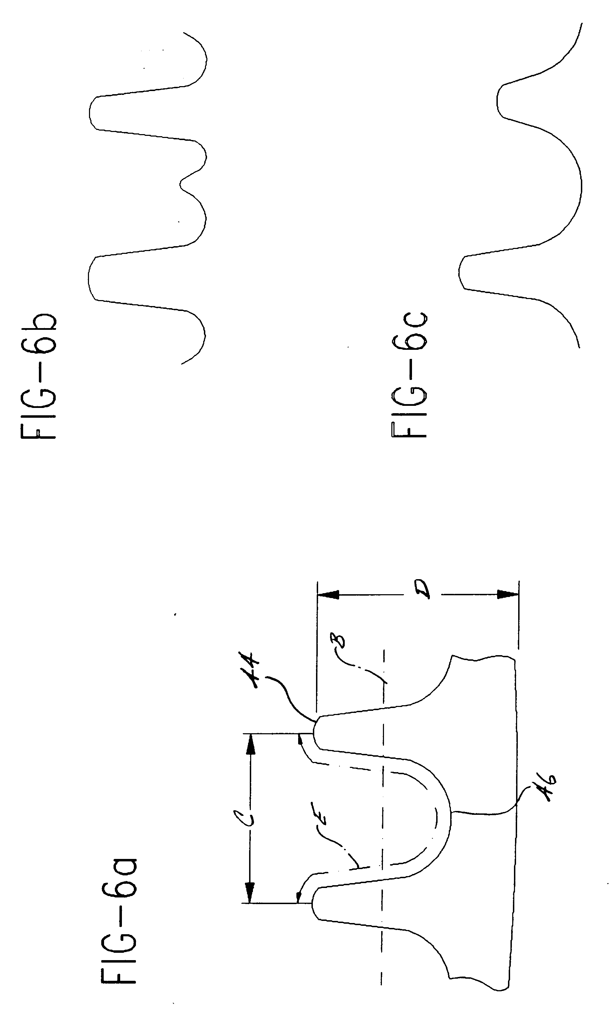

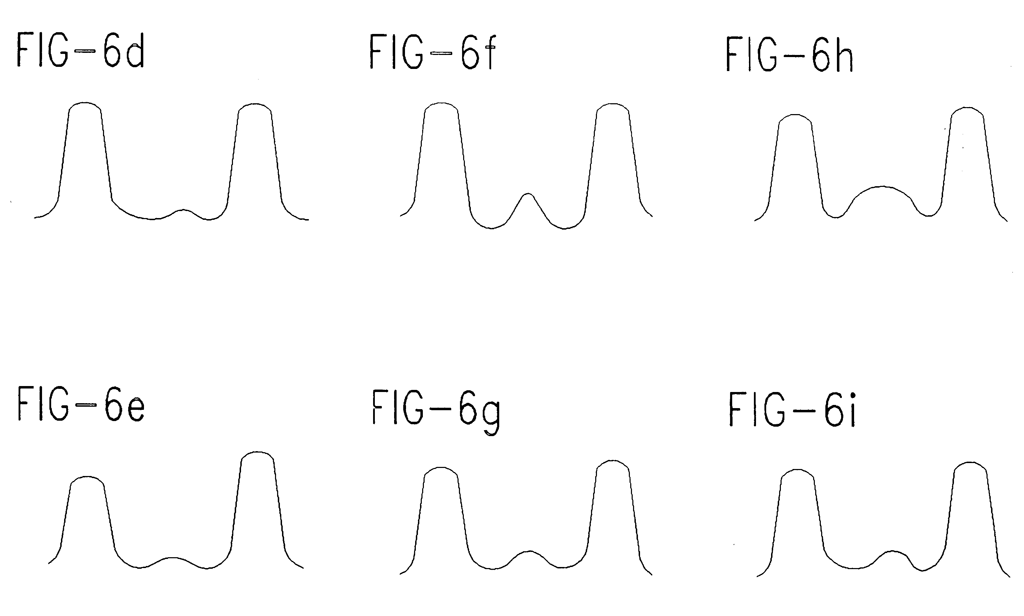

Experimental extrusions of sidewall portion 14 tubing were performed where several different designs of longitudinal elements 30 were integrally formed in bands on inner surface 26 of the sidewall by modifications to the mandrel of the extruder die. These variations are illustrated as line drawings taken from photo-micrographs of cross-sections in Figs. 6a-6i. All dimensions B and C are within the preferred ranges given hereinabove, the only variation being in a ratio R which is a contour path length E divided by the linear pitch distance C. Ratio R is a measure of the increase in surface area of inner surface 26 over a bottle with a substantially smooth surface. In bottles with a substantially smooth inner surface, as represented by current commercially available bottles, ratio R would equal 1.0. In the present invention, Ratio R is preferably about 1.5 to about 4 and most preferably is above about 2.

The results of the extrusion experiment showed that while variations in the design of mandrel did produce different shapes of longitudinal elements 30 on surface 26, the extrusion process requires relative uniformity of surface contour and uniform wall thickness D, for satisfactory extrusion. Ratio R and wall thickness D for the designs from the extrusion experiment illustrated in Figs. 6a-6i are given in Table 1.

**Table 1**

| Design | Ratio R | Wall Thickness D(mm) |
|---|---|---|
| 6a | 1.87 | 2.2 |
| 6b | 1.65 | 1.7 |
| 6c | 1.81 | 1.1 |
| 6d | 1.72 | 2.2 |
| 6e | 1.47 | 1.5 |
| 6f | 1.92 | 2.2 |
| 6g | 1.86 | 1.2 |
| 6h | 2.20 | 2.2 |
| 6i | 1.87 | 1.8 |

Cell culture experiments were run in bottles formed with sidewall portion 14 tubing from the extrusion experiment to characterize the ability of cells to grow in roller bottles on bands of designs as illustrated in Figs. 6a-6g in comparison to standard commercial roller bottles where inner surface 26 of sidewall portion 14 is substantially smooth and parallel to outer surface 28. Both experimental (Figs. 6a-6g) bottles and standard (substantially smooth bottles) were each charged with about ten million MRC-5 human embryonic lung fibroblast cells in about 200 ml of Eagle's minimum essential medium containing non-essential amino acids (MEM/NEAA) supplemented with 10 percent fetal bovine serum (FBS). The bottles were placed on a standard rolling apparatus and rotated at 1 rpm (with caps loosened to promote gas exchange) for 10 days at 37°C in a 5 percent carbon dioxide atmosphere. The medium was drained and replaced on days 3, 6, and 9. The results on day 10, of a colorimetric assay based on thiazolyl blue tetrazolium salt (MTT) with cleavage by mitochondrial dehydrogenases that is usually interpreted as relative cell number, showed a statistically significant 78 percent increase in relative cell numbers for the bottle having multiple longitudinal elements 30 when compared to the standard smooth surface bottle.

Bottles having designs as illustrated in Figs. 6a-6g with longitudinal elements in bands on inside surface 26 and smooth-walled control bottles each were charged with about ten million MRC-5 cells in about 200 ml of complete medium (MEM/NEAA supplemented with 10 percent FBS). The bottles were placed on laboratory rollers and rotated at 1 rpm (with caps loosened) for 13 days in an atmosphere of 5 percent carbon dioxide at 37°C. The medium was drained and replaced on days 2 and 8. On day 13, the drained bottles were longitudinally sectioned to isolate individual design bands, and the individual sections were extracted with an aqueous alkaline detergent (sodium dodecyl sulfate [SDS]). These extracts were evaluated with a modified Lowry colorimetric assay for total protein, which can be interpreted as a relative measure of total cell number. The results show a statistically significant average 68 percent increase in numbers of cells for the presently invented bottles over the smooth control bottles with a range from about 64 percent to 86 percent for the individual section designs. Each of the individual section designs as illustrated in Figs. 6a-6g were statistically significantly different from the smooth control, but were not statistically different from each other.

Bottles having designs as illustrated in Figs. 6a-6g with longitudinal elements in bands on inside surface 26 and smooth-walled control bottles each were charged with about twenty million Chinese hamster ovary cells (CHO-K1) in about 200 ml of a 1:1 mixture of Dulbecco's modified Eagle's medium and Ham's F12 medium(DME/F12) supplemented with 5 percent FBS. The bottles were place on standard roller apparatus and rotated (with loosened caps) at 1 rpm for 11 days in an atmosphere of 5 percent carbon dioxide at 37°C. The medium was drained and replaced at days 4 and 8. On day 11, the drained bottles were longitudinally sectioned, and the individual sections were extracted with aqueous alkaline SDS. These extracts were assayed with the modified Lowry assay for total protein as a relative measure of total cell number. The results showed a statistically significant 41 percent increase for the Figs. 6a-6g bottles over the smooth surface control bottles with a range from about 33 to 63 percent for individual section designs. Each of the individual section designs as illustrated in Figs. 6a-6g were statistically significantly different from the smooth control, but were not statistically different from each other.

Based on these findings, the most preferred embodiment was selected for extrudability. The most preferred embodiment has elevations about 0.6mm above (first portion 44) and about 0.2mm below (second portion 46) average surface elevation B, the C (pitch or distance between like elements) dimension of about 1.1mm and the D (wall thickness) dimension of about 2.2mm. The shape of the contour path (E) is determined by the need to maintain soft transitions and gentle curves, typically resembling Figs. 3 and 6h. The most preferred embodiment has ratio R (ratio of the length of the contour path to the pitch) of about 2.2.

Sidewall portion 14 tubing sections were extruded using the most preferred dimensions and the most preferred contour as shown in Figs. 3 and 6h. Roller bottles then were assembled using these sections where the inside surface 26 had 313 longitudinal elements spaced at a pitch of about 1.1mm. The balance of interior surface 26 was occupied by substantially optically transparent element 50 for viewing the cells. Bottles with these most preferred sidewall sections were submitted for cell culture testing against standard bottles having a smooth interior surface. Both the most preferred embodiment bottles and the smooth inner surface controls were charged with about ten million MRC-5 human lung fibroblast cells in 200ml MEM/NEAA supplemented with 10 percent FBS. Charged bottles with loosened caps were rotated at 1 rpm at 37°C in a 5 percent CO₂ atmosphere for 9 days. The culture medium was drained and replaced at day 3 and day 6. At day 9, medium was drained and a total cell extract was made from each bottle with aqueous alkaline SDS. This extract was assayed for protein using the modified Lowry colorimetric method. The most preferred embodiment of the instant invention having a multiplicity of longitudinal elements 30 showed a statistically significant 45 percent increase in cell growth over the standard commercial smooth side control.

When the present invention is compared in usage to the prior art represented by United States Patent 5,010,013 and related patents, bottles of the present invention were charged with only 200ml of growth medium and achieved full length coverage of a portion of the surface. Bottles as taught by '013, with pleats perpendicular to the longitudinal axis which are large compared to the wall thickness, require 450 to 500ml of media to ensure full length coverage of the pleats. Additionally, since the pleat height taught by '013 is on the order of a centimeter rather than the present invention with about 0.1 to about 1.3mm longitudinal elements above and below the average inside surface elevation, splashing, which would occur during drainage and refill, may more easily dislodge cells from the surface of the '013 bottle. Further, if the desired product from the cell culture is released to the growth medium, larger volumes, hence more dilute solutions, for concentration and recovery of the desired product result with the '013 bottle.

The comparative tests presented hereinabove demonstrate the benefits of the present invention over the currently commercial smooth sided bottle and the art as represented by bottles having large axially perpendicular pleats. A further benefit to the present invention is that it can be produced on existing equipment at comparable efficiencies by simply replacing the central extrusion mandrel. The art of cell culturing in roller bottles is advanced by the present invention.

## Claims

1. A roller bottle assembly for cell growth culturing comprising:
a generally cylindrical bottle with a longitudinal axis having a bottom portion, a sidewall portion having a first end and a second end, and a top portion, said portions defining a hollow chamber, said bottom portion being fluidly tightly attached to said sidewall portion at said first end generally perpendicular to said axis, said top portion being fluidly tightly attached to said second end of said sidewall portion generally perpendicular to said axis and having means for introducing liquid into said chamber; and
said sidewall portion having an inner surface and an outer surface, said inner surface having a multiplicity of longitudinal elements integrally formed thereon serving as attachment surfaces for growing cells.

2. The roller bottle assembly of claim 1 wherein said means for introducing liquid comprises a neck portion with an opening to said chamber having a removable conjugate cap for sealing said opening.

3. The roller bottle assembly of claim 1 wherein said bottom portion and said top portion have means for positioning said bottle on a roller rack system; and
said bottom portion having an axial concave section positioned to project into said chamber so that an axially positioned neck portion with a removable cap of a similar bottle placed adjacent to said bottle on the roller rack system can project into said chamber area, thereby conserving space on the roller rack system.

4. The roller bottle assembly of claim 1, wherein said inner surface further has a surface treatment selected from the group consisting of plasma treatment, corona discharge treatment, and surface oxidation to facilitate cell attachment and growth.

5. The roller bottle assembly of claim 1 wherein said top portion and said bottom portion are fluidly tightly attached to said sidewall portion by a method selected from the group consisting of adhesive bonding, solvent bonding, spin welding, ultrasonic welding and mechanical interference fitting.

6. The roller bottle assembly of claim 1 wherein said bottle is formed from a plastic resin.

7. The roller bottle assembly of claim 6 wherein said plastic resin is a thermoplastic selected from the group consisting of polypropylene, polycarbonate, polyurethane, polyvinyl chloride, polymethylmethacrylate, polystyrene, polyethyleneterephthalate and polyester with said forming including extrusion of said sidewall portion.

8. The roller bottle assembly of claim 1 wherein said longitudinal elements include a first portion having an elevation above an average surface elevation of said inside surface, a second portion having an elevation below said average surface elevation of said inside surface, said longitudinal elements further including longitudinal substantially smooth transition areas between said first and second portions.

9. The roller bottle assembly of claim 8 wherein said longitudinal elements further include at least one substantially optically transparent portion positioned to allow inspection of cells on said inner surface from outside said bottle.

10. The roller bottle assembly of claim 8 wherein said first portion and said second portion with said substantially smooth transitions therebetween, are sized to facilitate cell attachment and growth, said first portion and said second portion having elevations with a range of about 1.3mm to about 0.1mm above and below said average elevation of said surface, and a pitch range between like elements of about 0.5mm to about 1.6mm.
